# EUROPEAN PATENT APPLICATION

(11) **EP 1 611 899 A1**
(43) Date of publication of application: **04.01.2006**
(21) Application number: 04722061.1
(22) Date of filing: 19.03.2004
(51) Int. Cl.: A61K 38/16

(54) **COMBINED USE OF G-CSF WITH ANGIOGENETIC FACTOR**

(30) Priority: 20.03.2003 JP 2003078749; 13.02.2004 JP 2004036050
(71) Applicant: Fukuda, Keiichi, Tokyo 1760006 (JP); Hisaka, Yasuyo, Tokyo 151-0051 (JP)
(72) Inventor: Fukuda, Keiichi, Tokyo 1760006 (JP); Hisaka, Yasuyo, Tokyo 151-0051 (JP)
(74) Representative: Vossius & Partner
(86) International application number: PCT/JP2004/003790
(87) International publication number: WO 2004/082703

(57) **Abstract**

An effective remedy for ischemic disease, which contains granulocyte colony-stimulating factor (G-CSF) and hepatocyte growth factor (HGF) or fibroblast growth factor (FGF) as active ingredients, is disclosed. By administering this remedy, an effective therapy particularly for obstructive arteriosclerosis is provided which can eliminate drawbacks with conventional therapies such as kinesitherapy, pharmacotherapy, revascularization and recently proposed therapies such as gene therapy and intramuscular inoculation of bone marrow cells. Furthermore, the remedy of the present invention can become a remedy for ischemic disease such as ischemic cerebrovascular disorder or ischemic heart disease.

## Description

### TECHNICAL FIELD

The present invention relates to a remedy for ischemic disease, i.e., a composition for treating ischemic disease, which comprises granulocyte colony-stimulating factor (G-CSF) and a factor having an angiogenic action as active ingredients.

### BACKGROUND ART

The present invention is an invention concerned with remedies for ischemic disease. One typical ischemic disease, obstructive arteriosclerosis, will be described first.

Obstructive arteriosclerosis is a disease in which an arteriosclerotic (atherosclerotic) lesion results in deposition of an atheromatous substance mainly consisting of fats on the endarterium, to arouse occlusion or stenosis of a major truncal artery in the extremity, especially in the lower limb, thereby causing an ischemic disorder in its periphery. Clinical symptoms of this disease are classified as coldness or numbness, intermittent claudication, rest pain, and ulcer/necrosis. In Japan, patients with obstructive arteriosclerosis are estimated to number about 100,000 (Yusuke Tada: Biomedicine & Therapeutics, Vol. 31, 289-292; 1997). The number of patients with this disease is expected to increase because of the increase in the elderly population and the westernization of diets.

Therapies of obstructive arteriosclerosis include kinesitherapy or exercise therapy, pharmacotherapy, and revascularization, which are selected depending on symptoms or the patient's condition. Other measures, now under consideration, for avoiding a resection of a severely ischemic limb are angiogenic therapies (gene therapy, bone marrow autotransplantation, etc.) for promoting angiogenesis. These therapies are currently achieving some success in the treatment of obstructive arteriosclerosis, but the respective therapies involve the following problems.

In some mild cases, the distance of walking has increased in exercise therapy. However, the effect of this therapy is difficult to predict. Moreover, patients are not satisfied with the increase in the walking distance, if any, and 30% of them are reported to have requested revascularization (Takashi Ohta: Japan Medical Journal, Vol. 3935, 25-29, 1999). Thus, at present, this therapy is not a very effective form of treatment.

In pharmacotherapy, antiplatelet agents are mainly prescribed, but they merely prevent an aggravation of symptoms. Microcirculation improving agents and oxygen transport improving agents, which have recently been developed aggressively, are only expected to be indicated for mild cases. Nowadays, there are no radical remedies available for obstructive arteriosclerosis.

Revascularization, on the other hand, is currently the most effective therapy, which involves percutaneous angioplasty or a bypass operation depending on the condition of the patient or the location or extent of the lesion. However, these surgical operations are so extensive that they pose problems, such as surgery-associated complications or death, and a poor prognosis for a long life.

Gene therapy using angiogenic factor is aimed at correcting ischemia by developing collateral circulation channels. Examples of known angiogenic factors are vascular endothelial growth factor (VEGF), epidermal growth factor (EGF), hepatocyte growth factor (HGF), and fibroblast growth factor (FGF). In Japan, clinical studies using human HGF are under way. A method, which involves its intramuscular injection into the lower limb muscle using a plasmid carrying HGF gene, has been investigated in patients with severely ischemic limbs, and expectations are growing for its efficacy. However, this therapy is still at the experimental stage, and evaluations of its safety and efficacy have not been fully carried out. Thus, gene therapy has not become popular.

Intramuscular transplantation of autologous bone marrow cells, which has recently attracted attention, is a therapy in which bone marrow cells are transplanted into the muscle near the diseased part, whereafter they are differentiated into vascular endothelial cells to form blood vessels, thereby treating the diseased part. Bone marrow autotransplantation has no adverse effects on the immune system, and has been recognized to present differentiation of bone marrow cells into endothelial cells or increase the number of blood vessels in animal models. Although its efficacy will have to be evaluated in an increased number of patients, this therapy is expected to become a promising one, because it can treat severe cases. However, the bone marrow is taken under general anesthesia in a clinical setting, so the heavy burden imposed on the patient and medical staff in taking the bone marrow may present problems.

Recent studies have shown that hematopoietic stem cells, which can differentiate into vascular endothelial cells, are present not only in the bone marrow, but also in the peripheral blood, and they take part in angiogenesis (Qun Shi et al., Blood vol. 92, 362-367, 1998; Takayuki Asahara et al., Circulation Research vol. 85, 221-228, 1999; Mario Peichev et al., Blood vol. 95, 952-958, 2000). (The hematopoietic stem cells are called "precursor cells for endothelial cells" from the viewpoint of the function of differentiating into endothelial cells. However, these cells are originally derived from hematopoietic stem cells. Thus, the term "hematopoietic stem cells" is used herein in accordance with the concept that they are a cell population capable of becoming endothelial cells.) Hence, hematopoietic stem cells in the peripheral blood are taken and transplanted into the muscle close to the diseased part, whereby treatment of obstructive arteriosclerosis can be expected. This procedure is advantageous in that the burden imposed on the patient and medical staff at the time of taking peripheral blood stem cells is less than that during transplantation of stem cells present in the bone marrow. Normally, however, the frequency of existence of hematopoietic stem cells in the peripheral blood is extremely low. Thus, it is highly questionable whether a necessary and adequate amount of hematopoietic stem cells for the treatment of obstructive arteriosclerosis can be obtained.

Human G-CSF is a hematopoietic factor discovered as a differentiation/growth factor for progenitor cells of the granulocytic lineage. It is clinically applied as a remedy for neutropenia following bone marrow transplantation or cancer chemotherapy, because it facilitates neutrophilic hematopoiesis in vivo. In addition to this action, human G-CSF acts on hematopoietic stem cells to stimulate their proliferation and differentiation, and also acts to mobilize hematopoietic stem cells present in the bone marrow into the peripheral blood. Actually, based on the latter action, transplantation of the peripheral blood hematopoietic stem cells mobilized by human G-CSF, i.e. peripheral blood stem cell transplantation, is performed in the clinical setting, with the aim of accelerating hematopoietic recovery in cancer patients after intensive chemotherapy. This hematopoietic stem cell mobilizing action of G-CSF is far more potent than that of GM-CSF, also a hematopoietic factor for the granulocytic lineage. In terms of few side effects as well, G-CSF has superiority over GM-CSF.

HGF is a protein which is produced by various mesenchymal cells and targets many epithelial cells, neurons, endothelial cells, and some mesenchymal cells. HGF is known to have cell motility promoting activity and epithelial morphogenesis (luminal structure, etc.) inducing activity, in addition to cell proliferation promoting activity. Since HGF functions as an organ regenerating factor for promoting the regeneration of the kidney, the lung and the digestive tract, as well as the liver, in adults, it is expected to be a remedy for organ disease.

### DISCLOSURE OF THE INVENTION

In patients with obstructive arteriosclerosis, administration of G-CSF prior to treatment with intramuscular transplantation of bone marrow cells can be expected to increase the frequency of hematopoietic stem cells in the bone marrow. Thus, the number of bone marrow punctures for collecting bone marrow cells can be reduced, and the burden on the patient can be reduced. On this occasion, the burden on the patient and the medical staff can be further reduced by obtaining hematopoietic stem cells for transplantation from the peripheral blood. Furthermore, hematopoietic stem cells in the peripheral blood have been shown to contribute to vasculogenesis, so that the increase of hematopoietic stem cells in the peripheral blood induced by the administration of G-CSF is speculated to promote vasculogenesis. Hence, the mere administration of G-CSF to patients can be expected to treat obstructive arteriosclerosis. This treatment for obstructive arteriosclerosis by the administration of G-CSF will clearly reduce the burden on the patient and the medical staff markedly in that it obviates the need for collection and transplantation of hematopoietic stem cells.

Besides, the combined use of G-CSF and gene therapy using angiogenic factor is expected to enhance the therapeutic effect. That is, G-CSF is caused to act on hematopoietic stem cells, stimulating their proliferation and differentiation. Also, hematopoietic stem cells in the bone marrow are mobilized into the peripheral blood to promote vasculogenesis. At the same time, angiogenesis is promoted by HGF or FGF. Effective utilization of these different actions can be predicted to show an additive or synergistic angiogenic effect.

Treatment for obstructive arteriosclerosis using G-CSF can be expected to take effect in severe cases, and will be of great benefit to patients. If this treatment is combined with treatment with an angiogenic factor which promotes differentiation and growth of vascular endothelial precursor cells, such as vascular endothelial growth factor (VEGF), epidermal growth factor (EGF), hepatocyte growth factor (HGF) or fibroblast growth factor (FGF), or with the gene therapy of these factors, the therapeutic effect of that treatment is expected to be augmented further. In this case, these factors or their genes can be administered to patients, for example, at sites near the diseased part. Similarly, G-CSF is expected to show an increased therapeutic effect, when combined with agents clinically used as drug therapies for obstructive arteriosclerosis, such as antiplatelet agents, vasodilators, microcirculation improvers, anticoagulants and antilipemic agents.

As a result of the foregoing analyses, the inventors of the present invention have found that G-CSF, when administered in combination with HGF or FGF as an angiogenic factor, produces a particularly significant improving effect on ischemic blood vessels, and thereby have accomplished the present invention.

Thus, the present invention provides a remedy for ischemic disease, which comprises G-CSF and HGF or FGF as active ingredients.

Furthermore, the remedy of the present invention is applicable as a remedy for the following diseases, similar ischemic diseases: trauma, rejection reaction during transplantation, ischemic cerebrovascular disorder (such as apoplexy or cerebral infarction), ischemic renal disease, ischemic pulmonary disease, infection-related ischemic disease, ischemic disease of limbs, and ischemic heart disease (such as ischemic cardiomyopathy, myocardial infarction or ischemic heart failure). That is, the present invention provides remedies for these diseases, which contain G-CSF and HGF or FGF as active ingredients.

The present invention also provides a remedy for obstructive arteriosclerosis.

The present invention further provides an agent for revascularization or muscle regeneration.

### BRIEF DESCRIPTION OF DRAWINGS

Figure 1 is a view showing the effects of administration of physiological saline (control), HGF, G-CSF and HGF+G-CSF, respectively, on the lower limb muscle weight ratio (%) in mice with an ischemic left paw.
Figure 2 is a view showing the effects of administration of physiological saline (control), HGF, G-CSF and HGF+G-CSF, respectively, on the lower limb blood flow ratio (%) in mice with an ischemic left paw.
Figure 3 is a view showing the effects of administration of physiological saline (control), HGF, G-CSF and HGF+G-CSF, respectively, on the blood flow rate in mice with an ischemic left paw. The red portion represents the highest flow rate, followed by the yellow, green and blue portions in decreasing order.
Figure 4A presents fluorescence photomicrographs showing the effects of administration of physiological saline (control: HGF-, G-CSF-), HGF plasmid (HGF+, G-CSF-), G-CSF (HGF-, G-CSF+) and G-CSF+HGF plasmid (HGF+, G-CSF+), respectively, on the ratio of GFP-positive cell numbers in mice with an ischemic left paw. GFP-positive cells are indicated in green and nuclei are indicated in blue.
Figure 4B is a graph showing scores for GFP-positive cell numbers calculated from the photomicrographs.
Figure 5A presents fluorescence photomicrographs showing the effects of administration of physiological saline (control: HGF-, G-CSF-), HGF plasmid (HGF+, G-CSF-), G-CSF (HGF-, G-CSF+) and G-CSF+HGF plasmid (HGF+, G-CSF+), respectively, on the ratio of vWF-positive cell numbers in mice with an ischemic left paw. vWF-positive cells are indicated in red and nuclei are indicated in blue. Figure 5B is a graph showing scores for vWF-positive cell numbers calculated from the photomicrographs.
Figure 6A shows fluorescence photomicrographs of GFP-positive cells (green), vascular endothelial cells (red) and nuclei (blue), as well as a merged image thereof.
Figure 6B shows fluorescence photomicrographs of GFP-positive cells (green), vascular smooth muscle cells (red) and nuclei (blue), as well as a merged image thereof.
Figure 7A shows fluorescence photomicrographs of GFP-positive cells (green), skeletal muscle cells (red) and nuclei (blue), as well as a merged image thereof. Figure 7B shows merged images of serial sections.

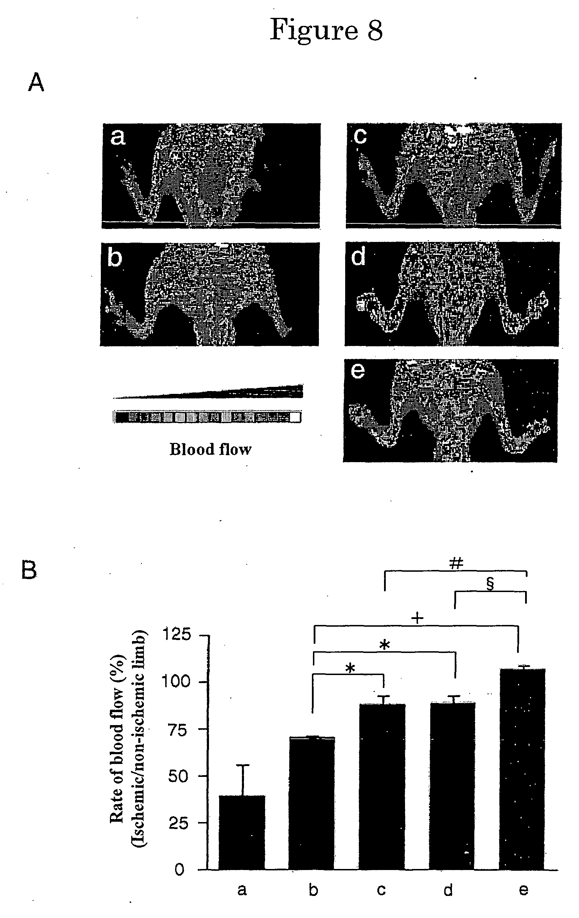

Figure 8 is a view showing the effects of administration of physiological saline (a: next day after surgery, b: 4 weeks after treatment), HGF plasmid (c: 4 weeks after treatment), G-CSF (d: 4 weeks after treatment) and G-CSF+HGF plasmid (e: 4 weeks after treatment), respectively, on the lower limb blood flow rate in mice with an ischemic left paw. The asterisk "*" denotes p<0.05 (vs. physiological saline treatment group) and the plus sign "+" denotes p<0.01 (vs. physiological saline treatment group). Likewise, the number mark "#" denotes p<0.05 (vs. HGF plasmid group) and the section mark "§" denotes p<0.05 (vs. G-CSF group).
Figure 9 is a graph showing scores for the degree of lower limb damage 4 weeks after treatment. White indicates no necrosis, gray indicates toe necrosis, and black indicates limb necrosis.
Figure 10 is a view showing the effects of administration of physiological saline, G-CSF, HGF plasmid, FGF, G-CSF+HGF plasmid and G-CSF+FGF, respectively, on the lower limb blood flow rate after 4 weeks in mice with an ischemic left paw. The asterisk "*" denotes p<0.05 (vs. physiological saline treatment group) and the dagger "t" denotes p<0.01 (vs. physiological saline treatment group). Likewise, the number mark "#" denotes p<0.05 (vs. G-CSF group), the section mark "$" denotes p<0.05 (vs. HGF plasmid group), and the paragraph mark "¶" denotes p<0.05 (vs. FGF group).
Figure 11 is a graph showing scores for the degree of lower limb damage 4 weeks after treatment. White indicates no necrosis, gray indicates toe necrosis, and black indicates limb necrosis.

### BEST MODE FOR CARRYING OUT THE INVENTION

The present invention relates to a remedy for ischemic disease, which comprises G-CSF and HGF or FGF.

As used herein, the term "ischemic disease" refers to a disease associated with local anemia caused by an organic disturbance in blood supply (e.g., arteriostenosis). Examples of ischemic diseases include trauma, rejection reaction during transplantation, ischemic cerebrovascular disorder (such as apoplexy or cerebral infarction), ischemic renal disease, ischemic pulmonary disease, infection-related ischemic disease, ischemic disease of limbs, and ischemic heart disease (such as ischemic cardiomyopathy, myocardial infarction or ischemic heart failure).

The present invention also relates to an agent for revascularization or muscle regeneration, which comprises G-CSF and HGF or FGF.

Human G-CSF is a known protein composed of 174 amino acid residues.

When G-CSF is used as the active ingredient of the remedy for ischemic disease according to the present invention, any type of G-CSF can be used, but highly purified G-CSF is preferred. Specific examples of G-CSF include mammalian G-CSF, especially human G-CSF, or G-CSF having substantially the same biological activity as mammalian G-CSF. The origin of G-CSF is not limited, and both naturally occurring G-CSF as well as G-CSF obtained by genetic recombination can be used. The G-CSF obtained by genetic recombination may be that having the same amino acid sequence as naturally occurring G-CSF (e.g., JP 1990-5395, JP 1987-236488 A), or that having this amino acid sequence subjected to deletion, substitution and/or addition of one or more amino acids, and having the same biological activity as naturally occurring G-CSF. For example, a polypeptide functionally comparable to G-CSF can be prepared by appropriately introducing a mutation into the amino acid sequence of G-CSF by use of such a method as site-directed mutagenesis (Gotoh, T. et al. (1995) Gene 152, 271-275; Zoller, M.J. and Smith, M. (1983) Methods Enzymol. 100, 468-500; Kramer, W. et al. (1984) Nucleic Acids Res. 12, 9441-9456; Kramer, W. and Fritz H.J. (1987) Methods Enzymol. 154, 350-367; Kunkel, T.A. (1985) Proc. Natl. Acad. Sci. USA, 82, 488-492; Kunkel (1988) Methods Enzymol. 85, 2763-2766). The mutation of an amino acid can occur in the natural world. It is already known that a polypeptide having a certain amino acid sequence modified by deletion and/or addition of one or more amino acid residues and/or substitution of an amino acid for the other amino acid retains the biological activity of the original polypeptide (Mark, D.F. et al., Proc. Natl. Acad. Sci. USA (1984) 81, 5662-5666; Zoller, M.L. & Smith, M. Nucleic Acids Research (1982) 10, 6487-6500; Wang, A. et al., Science (1984) 224, 1431-1433; Dalbadie-McFarland, G. et al., Proc. Natl. Acad. Sci. USA (1982) 79, 6409-6413).

Hence, a polypeptide comprising an amino acid sequence which has one or more amino acid mutations in G-CSF sequence, and being functionally equivalent to G-CSF, can also be used as the remedy for ischemic disease of the present invention. The number of amino acid mutations in such a polypeptide are normally within 30 amino acids, preferably within 15 amino acids, more preferably within 5 amino acids (for example, within 3 amino acids).

In the substitution mutant, substitution of an amino acid for the other amino acid which conserves the nature of the amino acid side chain is desirable. As the amino acid which conserves the nature of the amino acid side chain, there can be named, for example, hydrophobic amino acids (A, I, L, M, F, P, W, Y, V), hydrophilic amino acids (R, D, N, C, E, Q, G, H, K, S, T), amino acids having an aliphatic side chain (G, A, V, L, I, P), amino acids having a hydroxyl group-containing side chain (S, T, Y), amino acids having a sulfur atom-containing side chain (C, M), amino acids having a carboxylic acid- or an amide-containing side chain (D, N, E, Q), amino acids having a base-containing side chain (R, K,H), and amino acids having an aromatic-containing side chain (H, F, Y, W) (the symbols in the parentheses represent one-letter abbreviations for the corresponding amino acids).

Polypeptides in which a plurality of amino acid residues are added to the amino acid sequence of G-CSF include fusion polypeptides with G-CSF. Such fusion polypeptides are polypeptides produced by fusion between G-CSF and other polypeptide, and can also be used in the present invention. A fusion polypeptide can be prepared by, for example, ligating DNA coding for G-CSF with DNA coding for another polypeptide in-frame, transferring the resulting construct into a suitable expression vector, and expressing the insert in a suitable host. Other polypeptide to be fused to G-CSF is not limited as long as the resulting fusion polypeptide retains biological activity comparable to that of G-CSF.

Numerous reports are already present on G-CSF derivatives with the amino acid sequence of G-CSF changed, and thus these known G-CSF derivatives can be used (for example, USP 5,581,476, USP 5,214,132, USP 5,362,853 and USP 4,904,584).

Moreover, chemically modified G-CSF can be used. Examples of the chemically modified G-CSF include G-CSF subjected to conformational change, addition or deletion of the sugar chain, and G-CSF to which a compound such as polyethylene glycol has been bound (for example, USP 5,824,778, USP 5,824,784, WO 96/11953, WO 95/21629, WO 94/20069, USP 5,218,092, JP 1992-164098 A).

G-CSF in the present invention may be produced by any method. For example, it is possible to use G-CSF prepared by culturing a human tumor cell line, followed by extraction, isolation and purification by various methods, or G-CSF prepared by causing Escherichia coli; yeast; mammalian cells, such as Chinese hamster ovary cells (CHO cells), C127 cells, COS cells, myeloma cells or BHK cells; or insect cells to perform production by genetic engineering techniques, followed by extraction, isolation and purification by various methods (for example, JP 1989-44200, JP 1990-5395, JP 1987-129298 A, JP 1987-132899 A, JP 1987-236488 A and JP 1989-85098 A).

The method for producing this G-CSF may be any method which can give the product defined above. Concretely, the G-CSF is produced using G-CSF-producing tumor, G-CSF-producing hybridoma, or a transformed host which has been granted a G-CSF-producing potential by genetic recombination. Depending on the structure of G-CSF to be produced, a changing operation or various modifying operations are appropriately applied at a suitable stage of the production process. If the G-CSF is to be produced by genetic recombination, any routinely used host can be employed, such as Escherichia coli or animal cells.

In the present invention, G-CSF may be administered either as a protein or in the form of a gene coding for G-CSF, as in gene therapy.

HGF is a known heterodimeric protein comprising a 69 kDa α chain and a 34 kDa β chain.

The mode of administration of HGF is not limited, and HGF may be administered as a protein, but it is preferred to administer a gene coding for HGF, as in gene therapy. The gene coding for HGF is generally administered, for example, as an expression vector containing an expression cassette. The vector is not limited, and a non-virus vector may be used, or a virus vector may be used (e.g. Supplementary Volume of Experimental Medicine, "Experimental Methods for Gene Transfer and Expression Analysis," YODOSHA, 1997; Supplementary Volume of Experimental Medicine, "Basic Techniques for Gene Therapy", YODOSHA, 1996). Examples of the vector include a plasmid vector, a virus vector, a phage vector, a cosmid vector and a YAC vector. The expression vector normally includes a regulatory element, such as a promoter, and an antibiotic-resistance gene.

Any methods are available for gene transfer, and include, for example, calcium phosphate transfection, lipofection, a method using a liposome, the naked-DNA method, receptor-mediated gene transfer, a method using a gene gun, DEAE-dextran transfection, and a method using a capillary tube. In the present invention, the gene may be directly transferred into a body, or after gene transfer into cells taken up from the body, the cells may be returned into the body.

Since many reports have been issued on HGF and HGF expression vectors (HGF expression plasmids), those skilled in the art can appropriately select and administer them (e.g. Nakamura, T., Nishizawa, T., Hagiya, M. et al. Nature 1989, 342, 440-443; Hayashi, S., Morishita, R., Higaki, J. et al. Biochem Biophys Res Commun 1996, 220, 539-545; Morishita, R., Sakaki, M., Yamamoto, K. et al. Circulation, 2002, 105, 1491-1496). The administration of the gene encoding HGF can be performed by a method known to those skilled in the art (for example, WO 01/32220, WO 01/26694, WO 97/07824, WO 01/21214).

When HGF is administered as a protein, any type of HGF can be used, but highly purified HGF is preferred. Specific examples of HGF include mammalian HGF, especially human HGF, or HGF having substantially the same biological activity as mammalian HGF. The origin of HGF is not limited, and naturally occurring HGF and HGF obtained by genetic recombination can be used. The HGF obtained by genetic recombination may be that having the same amino acid sequence as naturally occurring HGF (e.g., GenBank Accession Nos.: M73239, M73240, M29145, L02931 and M60718), or that having this amino acid sequence subjected to deletion, substitution and/or addition of one or more amino acids, and having the same biological activity as naturally occurring HGF. For example, a polypeptide functionally comparable to HGF can be prepared by appropriately introducing a mutation into the amino acid sequence of HGF by use of such a method as site-directed mutagenesis (Gotoh, T. et al., 1995, Gene 152, 271-275; Zoller, M.J. and Smith, M., 1983, Methods Enzymol. 100, 468-500; Kramer, W. et al., 1984, Nucleic Acids Res. 12, 9441-9456; Kramer, W. and Fritz, H.J., 1987 Methods Enzymol. 154, 350-367; Kunkel, T.A., 1985, Proc. Natl. Acad. Sci. USA 82, 488-492; Kunkel (1988) Methods Enzymol. 85, 2763-2766). The mutation of an amino acid can occur in the natural world. It is already known that a polypeptide having a certain amino acid sequence modified by deletion and/or addition of one or more amino acid residues and/or substitution of an amino acid for the other amino acid retains the biological activity of the original polypeptide (Mark, D.F. et al., Proc. Natl. Acad. Sci. USA 81, 1984, 5662-5666; Zoller, M.L. & Smith, M., Nucleic Acids Res. 10, 1982, 6487-6500; Wang, A. et al., Science 224, 1984, 1431-1433; Dalbadie-McFarHand, G. et al., Proc. Natl. Acad. Sci. USA 79, 1982, 6409-6413).

Hence, a polypeptide comprising an amino acid sequence which has one or more amino acid mutation in HGF sequence, and being functionally equivalent to HGF, can also be used as a remedy for ischemic disease of the present invention. The number of amino acid mutations in such a polypeptide is normally within 30 amino acids, preferably within 15 amino acids, more preferably within 5 amino acids (for example, within 3 amino acids).

In the substitution mutants of HGF, substitution of an amino acid for the other amino acid which conserves the nature of the amino acid side chain is desirable, as in the case of G-CSF. Polypeptides in which a plurality of amino acid residues is added to the amino acid sequence of HGF include fusion polypeptides with HGF. Such fusion polypeptides are polypeptides produced by fusion between HGF and other polypeptide, and can also be used in the present invention. A fusion polypeptide can be prepared by, for example, ligating DNA coding for HGF with DNA coding for another polypeptide in-frame, transferring the resulting construct into a suitable expression vector, and expressing the insert in a suitable host. Other polypeptide to be fused to HGF is not limited as long as the fusion polypeptide retains biological activity comparable to that of HGF.

The gene coding for the HGF of the present invention includes a gene coding for such a polypeptide functionally equivalent to the HGF.

FGF encompasses acidic FGF (FGF1, 140 amino acids) and basic FGF (FGF2, 155 to 157 amino acids). Other functionally similar members include FGF3 (int-2), FGF4. (hst-1), FGF5, FGF6 (hst-2), FGF7 (KGF: keratinocyte growth factor), FGF8 (AIGF: androgen-induced growth factor) and FGF9 (GAF: Glia-activating factor). As used herein, the term "FGF" is intended to include all of them.

The mode of administration of FGF is not limited, and FGF may be administered either as a protein or in the form of a gene coding for FGF, as in gene therapy. The gene coding for FGF is generally administered, for example, as an expression vector containing an expression cassette. The vector is not limited, and a non-virus vector may be used, or a virus vector may be used (e.g. Supplementary Volume of Experimental Medicine, "Experimental Methods for Gene Transfer and Expression Analysis," YODOSHA, 1997; Supplementary Volume of Experimental Medicine, "Basic Techniques for Gene Therapy", YODOSHA, 1996). Examples of the vector include a plasmid vector, a virus vector, a phage vector, a cosmid vector and a YAC vector. The expression vector normally includes a regulatory element, such as a promoter, and an antibiotic-resistance gene.

Any methods are available for gene transfer, and include, for example, calcium phosphate transfection, lipofection, a method using a liposome, the naked-DNA method, receptor-mediated gene transfer, a method using a gene gun, DEAE-dextran transfection, and a method using a capillary tube. In the present invention, the gene may be directly transferred into a body, or after gene transfer into cells taken up from the body, the cells may be returned into the body.

Since many reports have been issued on FGF and FGF expression vectors (FGF expression plasmids), those skilled in the art can appropriately select and administer them (e.g., Kurokawa, T et al., FEBS Lett. 213, 189-194, (1987), GenBank Accession Nos.: J04513 and M27968). The administration of the gene encoding FGF can be performed by a method known to those skilled in the art (for example, Jejurikar S et al., Journal of Surgical Research, 67(2), 137-146, (1997), Reynolds P N et al., Tumor Targeting, 3(3), 156-168, (1998), Ruffini F et al., Gene Therapy, 8(16), 1207-1213, (2001)).

When FGF is administered as a protein, any type of FGF can be used, but highly purified FGF is preferred. Specific examples of FGF include mammalian FGF, especially human FGF, or FGF having substantially the same biological activity as mammalian FGF. The origin of FGF is not limited, and naturally occurring FGF and FGF obtained by genetic recombination can be used. The FGF obtained by genetic recombination may be that having the same amino acid sequence as naturally occurring FGF (e.g., FEBS Lett. 213: 189-194, 1987. GenBank Accession Nos.: J04513 and M27968), or that having this amino acid sequence subjected to deletion, substitution and/or addition of one or more amino acids, and having the same biological activity as naturally occurring FGF. For example, a polypeptide functionally comparable to FGF can be prepared by appropriately introducing a mutation into the amino acid sequence of FGF by use of such a method as site-directed mutagenesis (Gotoh, T. et al., 1995, Gene 152, 271-275; Zoller, M.J. and Smith, M., 1983, Methods Enzymol. 100, 468-500; Kramer, W. et al., 1984, Nucleic Acids Res. 12, 9441-9456; Kramer, W, and Fritz, H.J., 1987 Methods Enzymol. 154, 350-367; Kunkel, T.A., 1985, Proc. Natl. Acad. Sci. USA 82, 488-492; Kunkel (1988) Methods Enzymol. 85, 2763-2766). The mutation of an amino acid can occur in the natural world. It is already known that a polypeptide having a certain amino acid sequence modified by deletion and/or addition of one or more amino acid residues and/or substitution of an amino acid for the other amino acid retains the biological activity of the original polypeptide (Mark, D.F. et al., Proc. Natl. Acad. Sci. USA 81, 1984, 5662-5666; Zoller, M.L. & Smith, M., Nucleic Acids Res. 10, 1982, 6487-6500; Wang, A. et al., Science 224, 1984, 1431-1433; Dalbadie-McFarHand, G. et al., Proc. Natl. Acad. Sci. USA 79, 1982, 6409-6413).

Hence, a polypeptide comprising an amino acid sequence which has one or more amino acid mutations in FGF sequence, and being functionally equivalent to FGF, can also be used as the remedy for ischemic disease of the present invention. The number of amino acid mutations in such a polypeptide are normally within 30 amino acids, preferably within 15 amino acids, more preferably within 5 amino acids (for example, within 3 amino acids).

In the substitution mutants of FGF, substitution of an amino acid for the other amino acid which conserves the nature of the amino acid side chain is desirable, as in the case of G-CSF. Polypeptides in which a plurality of amino acid residues is added to the amino acid sequence of FGF include fusion polypeptides with FGF. Such fusion polypeptides are polypeptides produced by fusion between FGF and other polypeptide, and can also be used in the present invention. A fusion polypeptide can be prepared by, for example, ligating DNA coding for FGF with DNA coding for another polypeptide in-frame, transferring the resulting construct into a suitable expression vector, and expressing the insert in a suitable host. Other polypeptide to be fused to FGF is not limited as long as the fusion polypeptide retains biological activity comparable to that of FGF.

The gene coding for the FGF of the present invention includes a gene coding for such a polypeptide functionally equivalent to the FGF.

Moreover, chemically modified HGF or FGF can be used in the present invention. Examples of the chemically modified HGF or FGF include HGF subjected to conformational change, addition or deletion of the sugar chain, and HGF or FGF to which a compound such as polyethylene glycol has been bound.

HGF or FGF used in the present invention may be produced by any method. For example, it is possible to use HGF or FGF prepared by culturing a human tumor cell line, followed by extraction, isolation and purification by various methods, or HGF or FGF prepared by causing Escherichia coli; yeast; mammalian cells, such as Chinese hamster ovary cells (CHO cells), C127 cells, COS cells, myeloma cells or BHK cells; or insect cells to perform production by genetic engineering techniques, followed by extraction, isolation and purification by various methods. The method for producing this HGF or FGF may be any method which can give the product defined above. Concretely, the HGF or FGF is produced using a transformed host which has been granted an HGF or FGF-producing potential by, for example, genetic recombination. Depending on the structure of HGF or FGF to be produced, a changing operation or various modifying operations are appropriately applied at a suitable stage of the production process. If the HGF or FGF is to be produced by genetic recombination, any routinely used host can be employed, such as Escherichia coli or animal cells.

G-CSF, HGF and FGF are commercially available; it is also possible to use these commercially available products.

The remedy for ischemic disease according to the present invention can contain pharmaceutical carriers and vehicles necessary for assuming the form of a medicinal pharmaceutical composition, and can further contain stabilizers and adsorption preventing agents. Suitable dosage forms can be selected, including injections (such as subcutaneous injection, intradermal injection, intramuscular injection, intravenous injection and intraperitoneal injection), depot preparations, transnasal preparations, oral preparations (such as tablets, capsules, granules, liquids and solutions, and suspensions), transpulmonary preparations, transdermal preparations and transmucosal preparations. If desired, suitable devices can be used.

The remedy for ischemic disease according to the present invention can incorporate, if desired depending on the mode of its administration and its dosage form, a suspending agent, a solution adjuvant, a stabilizer, a tonicity agent, a preservative, an adsorption preventing agent, a surfactant, a diluent, an excipient, a pH regulator, a soothing agent, a buffering agent, a sulfur-containing reducing agent and an antioxidant.

Examples of the suspending agent are methylcellulose, polysorbate 80, hydroxyethylcellulose, acacia, tragacanth powder, sodium carboxymethylcellulose and polyoxyethylene sorbitan monolaurate.

Examples of the solution adjuvant are polyoxyethylene hydrogenated castor oil, polysorbate 80, nicotinamide, polyoxyethylene sorbitan monolaurate, macrogol and castor oil fatty acid ethyl ester.

Examples of the stabilizer are dextran 40, methylcellulose, gelatin, sodium sulfite and sodium metasulfite.

Examples of the tonicity agent are D-mannitol and sorbitol.

Examples of the preservative are methyl p-hydroxybenzoate, ethyl p-hydroxybenzoate, sorbic acid, phenol, cresol and chlorocresol.

Examples of the adsorption preventing agent are human serum albumin, lecithin, dextran, ethylene oxide-propylene oxide copolymer, hydroxypropylcellulose, methylcellulose, polyoxyethylene hydrogenated castor oil and polyethylene glycol.

Examples of the sulfur-containing agent are N-acetylcysteine, N-acetylhomocysteine, thioctic acid, thiodiglycol, thioethanolamine, thioglycerol, thiosorbitol, thioglycollic acid and its salts, sodium thiosulfate, glutathione and those having a sulfhydryl group such as a thioalkanoic acid having 1 to 7 carbon atoms.

Examples of the antioxidant are erythorbic acid, dibutylhydroxytoluene, butylhydroxyanisol, α-tocopherol, tocopheryl acetate, L-ascorbic acid and its salts, L-ascorbyl palmitate, L-ascorbyl stearate, sodium bisulfite, sodium sulfite, triamyl gallate; propyl gallate, and chelating agents such as disodium ethylenediaminetetraacetate (EDTA), sodium pyrophosphate and sodium metaphosphate.

The remedy for ischemic disease of the present invention may further contain normally added ingredients, such as inorganic salts, e.g., sodium chloride, potassium chloride, calcium chloride, sodium phosphate, potassium phosphate and sodium bicarbonate; and organic salts, e.g., sodium citrate, potassium citrate and sodium acetate.

The dose and the frequency of dosing of G-CSF contained in the remedy for ischemic disease according to the present invention can be determined in consideration of the condition of the patient for whom this remedy is indicated. The dose is usually 0.1 to 500 µg/kg/day, preferably 1 to 50 µg/kg/day, per adult. As the frequency of dosing, the remedy of the invention can be administered once to three times a day, for 1 to 7 days weekly. The mode of administration preferably includes intravenous administration, subcutaneous administration and intramuscular administration.

When G-CSF gene is given, its dose per adult is 0.1 µg to 100 mg, preferably 0.001 to 10 mg. When HGF gene is administered in the form of a liposome, its dose per adult is selected from the range of about 1 µg to about 4 mg, preferably the range of about 10 µg to about 400 µg.

In the present invention, when HGF gene is administered, choice is made of the mode of administration and the site of administration that are suitable for the disease and symptoms to be treated. The preferred site of administration is the muscle. The preferred mode of administration is the parenteral route.

The dose differs according to symptoms of the patient. When HGF gene is given, its dose per adult is 0.1 µg to 100 mg, preferably 0.001 to 10 mg. When HGF gene is administered in the form of a liposome, its dose per adult is selected from the range of about 1 µg to about 4 mg, preferably the range of about 10 µg to about 400 µg. The frequency of dosing is selected appropriately depending on symptoms of the patient. Preferably, the remedy is administered once in several days to several weeks, more preferably once weekly, totaling a plurality of times, further preferably a total of 8 times.

When HGF is administered as a protein, its dose and frequency of dosing can be determined in consideration of the condition of the patient for whom this remedy is indicated. The dose is usually 0.1 to 500 µg/kg/day, preferably 1 to 50 µg/kg/day, per adult. As the frequency of dosing, the remedy can be administered once to three times a day, for 1 to 7 days weekly. The mode of administration preferably includes intravenous administration, subcutaneous administration and intramuscular administration.

In the present invention, when FGF gene is administered, choice is made of the mode of administration and the site of administration that are suitable for the disease and symptoms to be treated. The preferred site of administration is the muscle. The preferred mode of administration is the parenteral route.

The dose differs according to symptoms of the patient. When FGF gene is given, its dose per adult is 0.1 µg to 100 mg, preferably 0.001 to 10 mg. When FGF gene is administered in the form of a liposome, its dose per adult is selected from the range of about 1 µg to about 4 mg, preferably the range of about 10 µg to about 400 µg. The frequency of dosing is selected appropriately depending on symptoms of the patient. Preferably, the remedy is administered once in several days to several weeks, more preferably once weekly, totaling a plurality of times, further preferably a total of 8 times.

When FGF is administered as a protein, its dose and frequency of dosing can be determined in consideration of the condition of the patient for whom this remedy is indicated. The dose is usually 0.1 to 500 µg/kg/day, preferably 1 to 50 µg/kg/day, per adult. As the frequency of dosing, the remedy can be administered once to three times a day, for 1 to 7 days weekly. The mode of administration preferably includes intravenous administration, subcutaneous administration and intramuscular administration.

However, the present invention is not limited by the doses of G-CSF and HGF or FGF. In the present invention, G-CSF and HGF or FGF can be prepared and administered as a single preparation. Alternatively, they can be prepared separately, and administered on different occasions.

By using the remedy for ischemic disease according to the present invention, the number of hematopoietic stem cells can be increased. The collection of these hematopoietic stem cells from the bone marrow or peripheral blood and their bone marrow autotransplantation to the patient himself or herself can contribute to vasculogenesis in peripheral blood, treating ischemic disease. The administration of the remedy according to the present invention also mobilizes hematopoietic stem cells into the peripheral blood, thus making it possible to treat ischemic disease, without collection or transplantation of hematopoietic stem cells.

Moreover, the remedy of the present invention can be combined with drugs hitherto used with expectation of effectiveness against ischemic disease, such as antiplatelet agents, vasodilators, microcirculation improvers, anticoagulants and antilipemic agents, and can also be used in combination with gene therapy.

The present invention will be described in more detail with reference to Experiments (pharmacological efficacy) and Examples (preparation examples), which in no way limit the present invention.

### EXAMPLES

### Experiment 1 (pharmacological efficacy)

(1) Wild type mice (C57BL/6, 8-10 weeks of age; CLEA, Tokyo, Japan) were irradiated once with a lethal dose of total body irradiation (850 cGy). Bone marrow cells (5 x 10⁶ cells) were collected from GFP transgenic mice (C57BL/6, 10-12 weeks of age) (Okabe et al., (1997) FEBS. Lett. 407, 313-319) and transplanted into the wild type mice through their tail veins. At 2 months after transplantation, the left femoral artery of each mouse was ligated at two locations to prepare lower limb ischemia models. These models were randomly divided into four groups, i.e., a physiological saline treatment group, a G-CSF treatment group, an HGF plasmid treatment group and a G-CSF+HGF plasmid treatment group (5 animals per group). HGF plasmid (Nakamura, T., Nishizawa, T., Hagiya, M. et al., Nature 1989, 342, 440-443; Hayashi, S., Morishita, R., Higaki, J. et al., Biochem Biophys Res Commun 1996, 220, 539-545; Morishita, R., Sakaki, M., Yamamoto, K. et al., Circulation 2002, 105, 1491-1496) was prepared using a plasmid purification kit (manufactured by QIAGEN) in accordance with the manufacturer's protocol. The physiological saline treatment group and the G-CSF (recombinant human G-CSF (Chugai Pharmaceutical Co., Ltd., Japan)) (300 µg/kg/day) treatment group received subcutaneous administration for 10 days, beginning 24 hours after ligature. The HGF plasmid treatment group received administration in a dose of 500 µg/animal by intramuscular injection performed 24 hours after ligature. The G-CSF+HGF plasmid treatment group received intramuscular injection of HGF (500 µg/animal) 24 hours after ligature and, immediately afterwards, received G-CSF treatment (300 µg/kg/day) for 10 days.

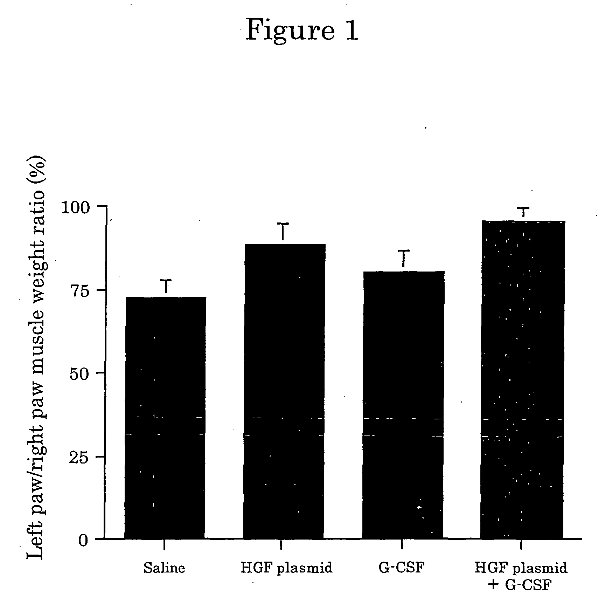

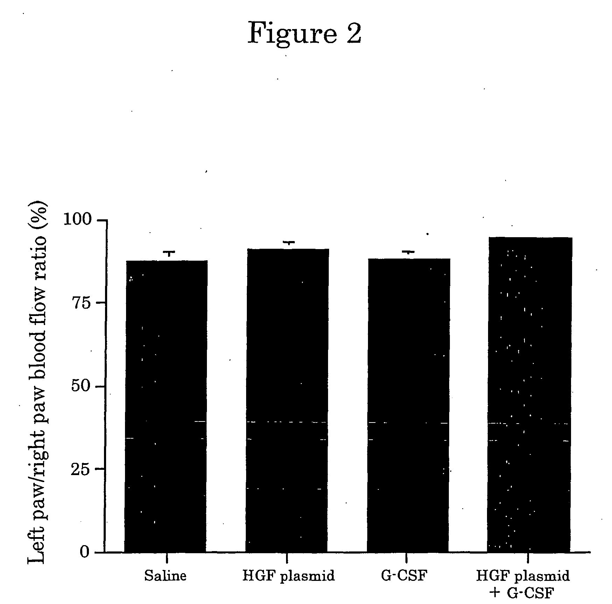

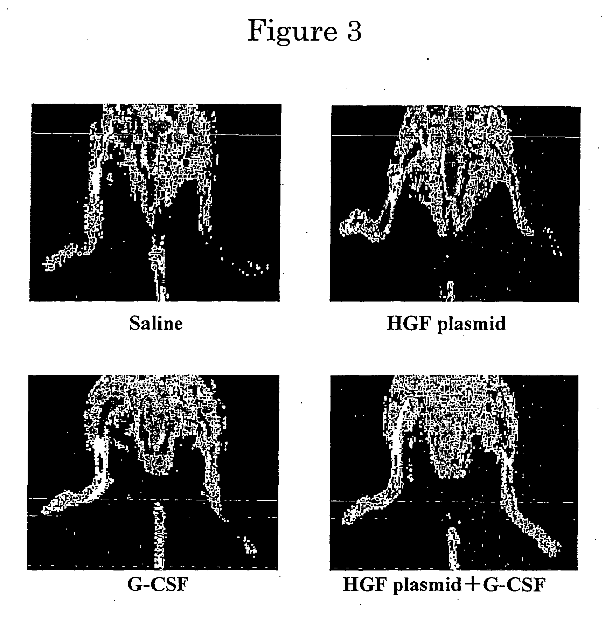

The drawings show the lower limb muscle weight ratio (Figure 1), the lower limb blood flow ratio (Figure 2), and the typical blood flow rate (Figure 3), 4 weeks after treatment, in each of the groups. The experimental data are shown in Table 1.

**Table 1**

| | Body weight (g) | | Lower limb muscle weight (g) | | Left foot/right foot muscle weight ratio (%) | Left foot/right foot blood flow ratio (%) |
|---|---|---|---|---|---|---|
| | Before experiments | After experiments | Right foot | Left foot | | |
| Physiological saline | 20.28±1.52 | 19.08±1.18 | 0.96±0.05 | 0.70±0.08 | 72.10±5.68 | 87.80±2.92 |
| HGF plasmid | 21.48±0.75 | 19.33±0.23 | 0.88±0.05 | 0.78±0.03 | 88.44±6.11 | 91.43±2.34 |
| G-CSF | 19.04±1.00 | 18.26±0.83 | 0.90±0.03 | 0.72±0.07 | 79.95±6.47 | 88.24±2.55 |
| HGF plasmid + G-CSF | 20.98±0.45 | 19.52±0.55 | 0.92±0.04 | 0.88±0.07 | 95.27±4.29 | 94.56±1.64 |

The G-CSF treatment group and the HGF plasmid treatment group showed a tendency toward improvement over the physiological saline treatment group. In the G-CSF+HGF plasmid treatment group, compared with the other groups, significant improvements were observed in the lower limb muscle weight ratio, the lower limb blood flow ratio and the blood flow rate, showing reduction of damage to the lower limb.

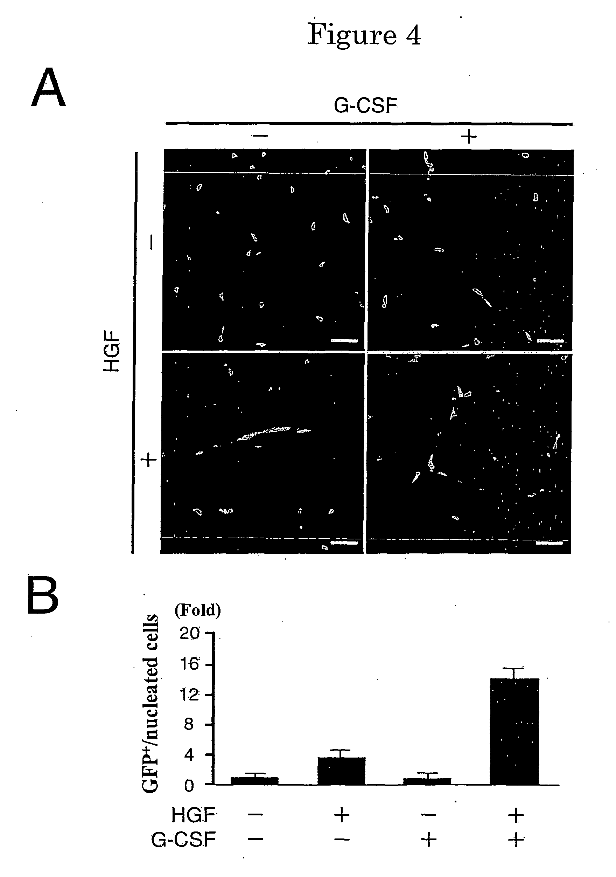

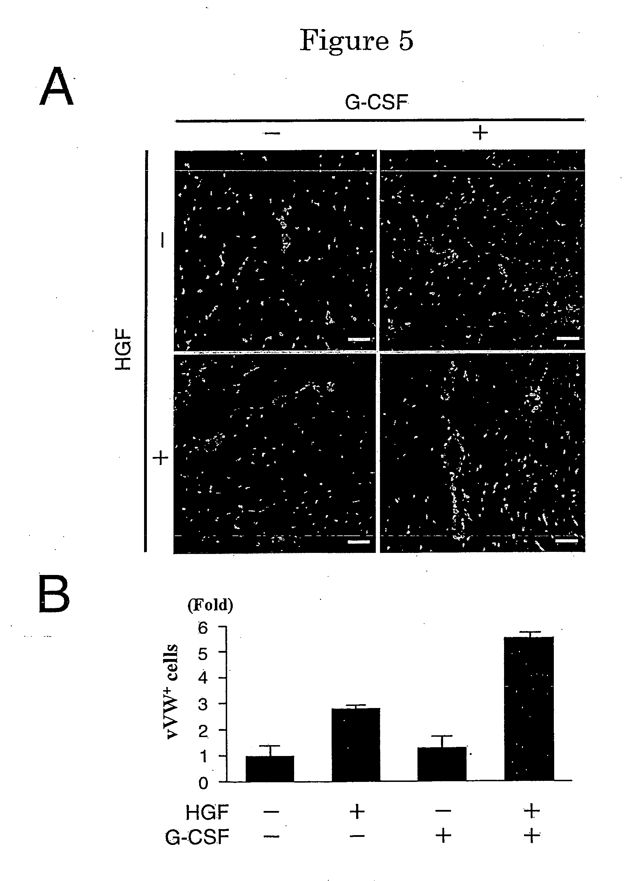

The above results suggested the combination of HGF and G-CSF to enhance a therapeutic effect as compared with HGF or G-CSF administered alone.
(2) Next, for histological observation, wild type mice were treated in the same manner as described above and then anesthetized with ketamine (30 mg/kg) and xylazine (6 mg/kg). Their lower limb blood vessels were perfused with PBS and fixed by perfusion of 4% paraformaldehyde in PBS. Ischemic lower limb muscle was excised, embedded in OCT compound (Miles Scientific, Naperville, IL, USA) and then rapidly frozen in liquid nitrogen to prepare sliced sections. The frozen sections (6 µm) were washed with PBS and stained overnight at 4°C using antibodies. The antibodies used for staining were: an anti-von Willebrand factor (vWF) antibody (clone F8/86; DAKO) for vascular endothelial cell staining; an α-smooth muscle actin antibody (clone 1A4; Sigma Aldrich) for vascular smooth muscle cell staining; and an anti-actinin antibody (clone EA-53; Sigma Aldrich) for skeletal muscle cell staining. The sections were then washed three times with PBS and incubated at 4°C for 4 hours in the presence of TRITC (DAKO, Japan)-labeled secondary antibody (red). The nuclei were stained in blue with TOTO-3 (Molecular Probes). The immunostained sections were observed under a confocal laser scanning microscope (LSM510META; Carl Zeiss, Jena, Germany) (Figures 4A and 5A).

Images obtained with the confocal laser scanning microscope were transferred to a computer and analyzed with NIH image software. GFP-positive cell numbers (Figure 4B) and vWF-positive cell numbers (Figure 5B) were calculated relative to nucleated cell numbers per section of the physiological saline treatment group (HGF-, G-CSF-).

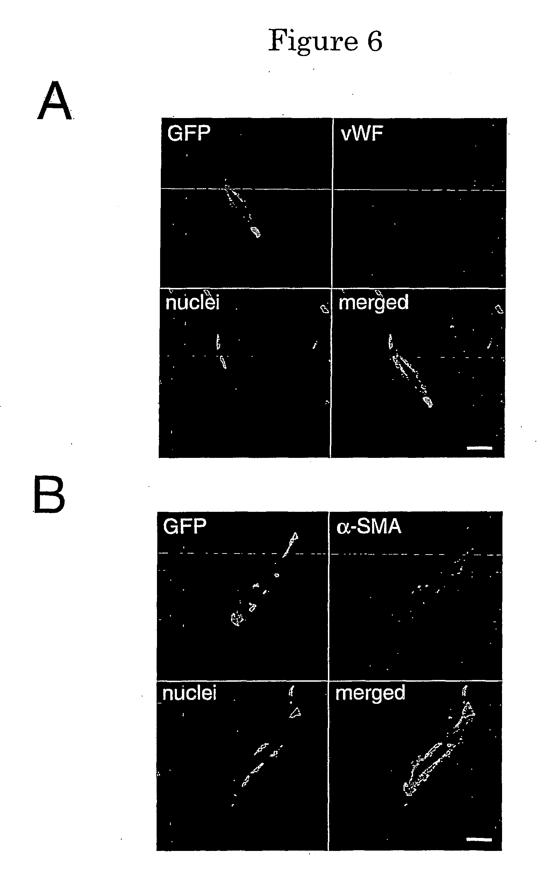

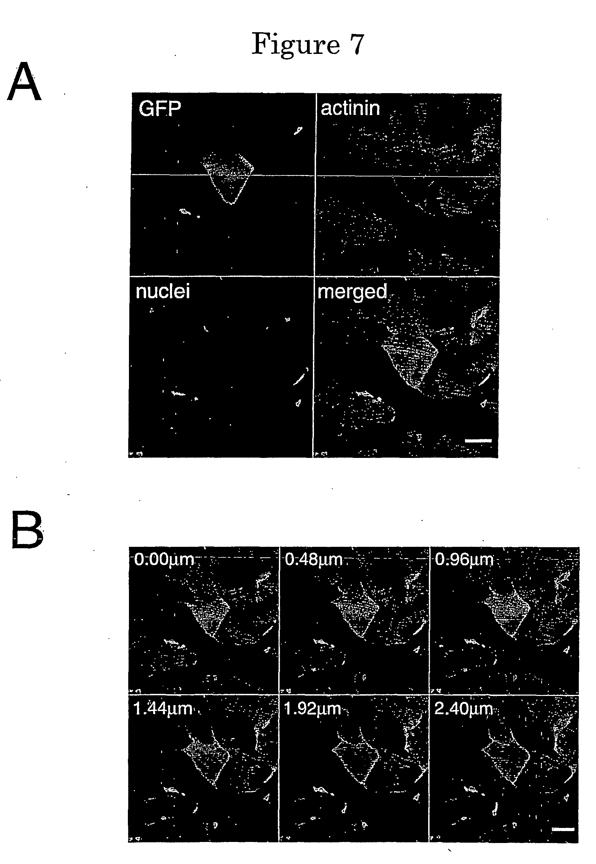

Further, Figure 6A shows a typical merged image of GFP-positive cells and vascular endothelial cells in serial sections, Figure 6B shows a merged image of GFP-positive cells and vascular smooth muscle cells, and Figure 7A shows a merged image of GFP-positive cells and skeletal muscle cells.

The immunostaining of lower limb muscle indicated that bone marrow cell-derived GFP-positive cells were differentiated into vascular smooth muscle cells and vascular endothelial cells. Although revascularization was enhanced even in the HGF plasmid treatment group as compared with the physiological saline treatment group, the G-CSF+HGF plasmid treatment group showed a synergistic effect on revascularization as compared with the other groups. The regenerated lower limb muscle derived from bone marrow cells was also observed. These results suggest that the combined therapeutic effect of G-CSF and HGF on lower limb damage is due to regeneration of blood vessels and/or lower limb muscle in ischemic limbs.

### Experiment 2 (pharmacological efficacy)

The left femoral artery of nude mice (BALB/cA) was ligated at two locations to prepare lower limb ischemia models. These models were randomly divided into four groups, i.e., a physiological saline group (20 animals), an HGF plasmid group, a G-CSF group and a G-CSF+HGF plasmid group (10 animals per group). In the same manner as shown in Experiment 1, the physiological saline treatment group and the G-CSF (300 µg/kg/day) treatment group received subcutaneous administration for 10 days, beginning 24 hours after ligature. The HGF plasmid group received administration in a dose of 500 µg/animal by intramuscular injection performed 24 hours postoperatively. The G-CSF+HGF plasmid treatment group received intramuscular injection of HGF (500 µg/animal) 24 hours postoperatively and, immediately afterwards, received G-CSF treatment (300 µg/kg/day) for 10 days. Figure 8 shows the typical blood flow rate, 4 weeks after treatment, in each of the groups. The blood flow rate was expressed as mean ± SEM, and statistical significance between mean values was calculated by ANOVA. Comparisons were made by log-rank test or non-parametric Fisher's multiple comparison test. The asterisk "*" denotes p<0.05 (vs. physiological saline treatment group) and the plus sign "+" denotes p<0.01 (vs. physiological saline treatment group). Likewise, the number mark "#" denotes p<0.05 (vs. HGF plasmid group) and the section mark "§" denotes p<0.05 (vs. G-CSF group).

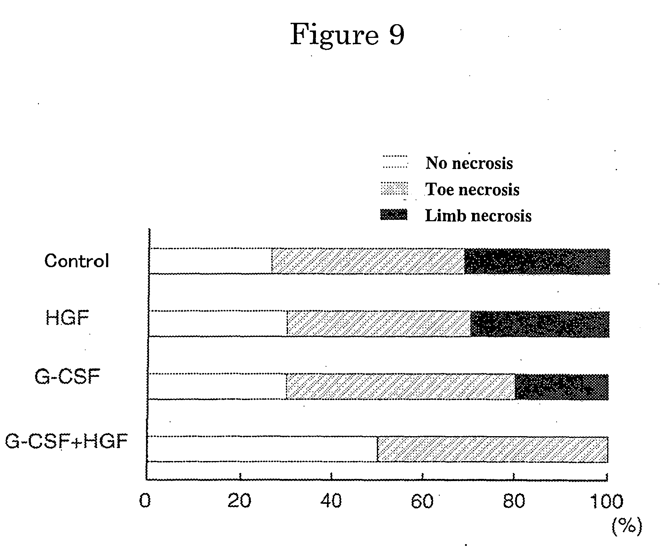

Moreover, the degree of lower limb damage 4 weeks after treatment was scored for evaluation (Figure 9). White indicates no necrosis, gray indicates toe necrosis, and black indicates limb necrosis.

Although even the groups treated with G-CSF or HGF plasmid alone showed a significant improvement in the lower limb blood flow over the physiological saline treatment group, the G-CSF+HGF plasmid group showed a more significant and synergistic effect on improving the lower limb blood flow as compared with the other groups. This effect had the same tendency as observed in the results of the wild type mice (Experiment 1), but it was found to be significantly higher in the nude mice than in the wild type mice. Likewise, the lower limb damage was also significantly reduced.

### Experiment 3 (pharmacological efficacy)

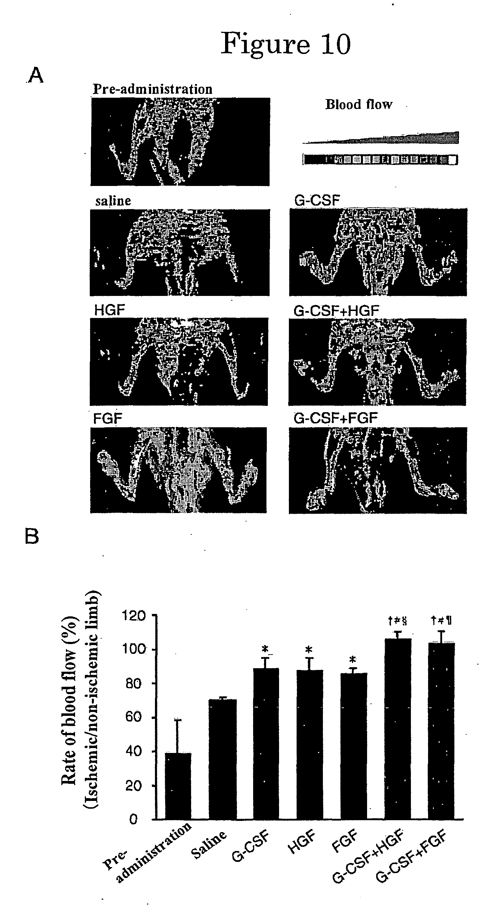

The left femoral artery of nude mice (BALB/cA) was ligated at two locations to prepare lower limb ischemia models. These models were randomly divided into six groups, i.e., a physiological saline group (20 animals), a G-CSF group, an HGF plasmid group, an FGF group, a G-CSF+HGF plasmid group and a G-CSF+FGF group (5 animals per group). In the same manner as shown in Experiment 1, the physiological saline treatment group and the G-CSF (300 µg/kg/day) treatment group received subcutaneous administration for 10 days, beginning 24 hours after ligature. The HGF plasmid group received administration in a dose of 500 µg/animal by intramuscular injection performed 24 hours postoperatively. The FGF group received intramuscular injection of FGF (500 µg/animal) (Trafermin: Kaken Pharmaceutical Co., Ltd., Japan) 24 hours postoperatively. The G-CSF+HGF plasmid treatment group received intramuscular injection of HGF (500 µg/animal) 24 hours postoperatively and, immediately afterwards, received G-CSF treatment (300 µg/kg/day) for 10 days. The G-CSF+FGF treatment group received intramuscular injection of FGF (500 µg/animal) 24 hours postoperatively and, immediately afterwards, received G-CSF treatment (300 µg/kg/day) for 10 days. Figure 10 shows the typical blood flow rate, 4 weeks after treatment, in each of the groups. The blood flow rate was expressed as mean ± SEM, and statistical significance between mean values was calculated by ANOVA. Comparisons were made by log-rank test or non-parametric Fisher's multiple comparison test. The asterisk "*" denotes p<0.05 (vs. physiological saline treatment group) and the dagger "t" denotes p<0.01 (vs. physiological saline treatment group). Likewise, the number mark "#" denotes p<0.05 (vs. G-CSF group), the section mark "§" denotes p<0.05 (vs. HGF plasmid group) and the paragraph mark "¶" denotes p<0.05 (vs.FGF group).

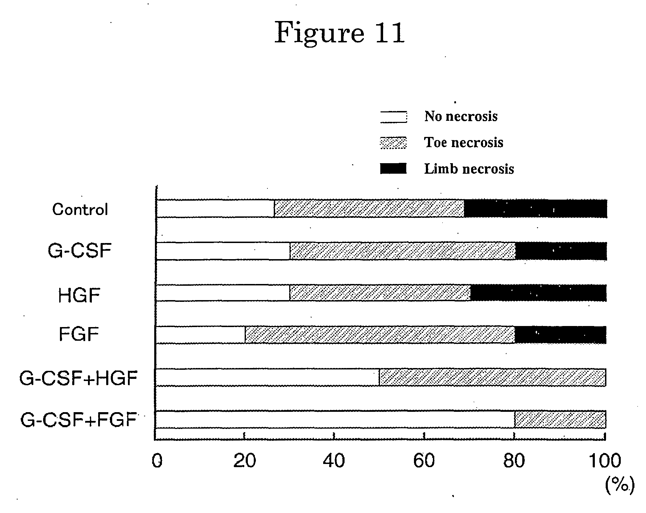

Moreover, the degree of lower limb damage 4 weeks after treatment was scored for evaluation (Figure 11). White indicates no necrosis, gray indicates toe necrosis, and black indicates limb necrosis.

Although even the groups treated with G-CSF or FGF alone showed a significant improvement in the lower limb blood flow over the physiological saline treatment group, the G-CSF+FGF group showed a more significant and synergistic effect on improving the lower limb blood flow as compared with the other groups. This effect had the same tendency as observed in the results of the wild type mice (Experiment 1), but it was found to be significantly higher in the nude mice than in the wild type mice. Likewise, the lower limb damage was also significantly reduced.

### Example 1 (preparation example)

Polysorbate 20 (Tween 20: polyoxyethylene sorbitan monolaurate), a nonionic surfactant, is added in an amount of 0.1 mg/ml to 50 µg/ml of human G-CSF (10 mM phosphate buffer, pH 7.0), and the mixture is adjusted to an osmotic pressure of 1 using NaCl. Then, the mixed solution is sterilized by filtration through a membrane filter having a pore size of 0.22 µm. The resulting solution is charged into a sterilized vial, whereafter the filled vial is capped with a similarly sterilized rubber stopper and then seamed with an aluminum cap to obtain a pharmaceutical solution for injection. This preparation for injection is stored in a cold dark place at 10°C or lower.

### Example 2 (preparation example)

Polysorbate 80 (Tween 80: polyoxyethylene sorbitan monooleate), a nonionic surfactant, is added in an amount of 0.1 mg/ml to 100 µg/ml of human G-CSF (10 mM phosphate buffer, pH 7.0), and the mixture is adjusted to an osmotic pressure of 1 using NaCl. Then, the mixed solution is sterilized by filtration through a membrane filter having a pore size of 0.22 µm. The resulting solution is charged into a sterilized vial, whereafter the filled vial is capped with a similarly sterilized rubber stopper and then seamed with an aluminum cap to obtain a pharmaceutical solution for injection. This preparation for injection is stored in a cold dark place at 10°C or lower.

### Example 3 (preparation example)

Polysorbate 20 (Tween 20: polyoxyethylene sorbitan monolaurate), a nonionic surfactant, in an amount of 0.1 mg/ml, 10 mg/ml of HAS and 50 mg/ml of mannitol are added to 50 µg/ml of human G-CSF (10 mM phosphate buffer, pH 7.0), followed by dissolving the mixture. Then, the solution is sterilized by filtration through a membrane filter having a pore size of 0.22 µm. The resulting solution is charged into a sterilized vial, whereafter the filled vial is half capped with a similarly sterilized rubber stopper and lyophilized to obtain a lyophilized preparation for injection. This lyophilized preparation for injection is stored under temperature conditions at room temperature or lower, and is dissolved, just before use, with distilled water for injection.

### INDUSTRIAL APPLICABILITY

The remedy for ischemic disease according to the present invention, which contains G-CSF and HGF or FGF as active ingredients, can be expected to show a therapeutic effect in relatively severe cases of obstructive arteriosclerosis, as demonstrated in Experiments 1 to 3. This effect of G-CSF and HGF or FGF is inferred to be based on the promotion of angiogenesis. Thus, this remedy can be expected to be therapeutically effective against other ischemic diseases, namely, trauma, rejection reaction during transplantation, ischemic cerebrovascular disorder (such as apoplexy or cerebral infarction), ischemic renal disease, ischemic pulmonary disease, infection-related ischemic disease, ischemic disease of limbs, and ischemic heart disease (such as ischemic cardiomyopathy, myocardial infarction or ischemic heart failure). The therapies according to the present invention are convenient, safe and efficacious as compared with conventional therapies.

## Claims

1. A remedy for ischemic disease, comprising granulocyte colony-stimulating factor and hepatocyte growth factor or fibroblast growth factor as active ingredients.

2. The remedy for ischemic disease according to claim 1, wherein the ischemic disease is trauma, rejection reaction during transplantation, ischemic cerebrovascular disorder, ischemic renal disease, ischemic pulmonary disease, infection-related ischemic disease, ischemic disease of limbs or ischemic heart disease.

3. The remedy for ischemic disease according to claim 1, wherein the ischemic disease is apoplexy, cerebral infarction, ischemic cardiomyopathy, myocardial infarction, ischemic heart failure or obstructive arteriosclerosis.

4. The remedy for ischemic disease according to claim 1, wherein the ischemic disease is obstructive arteriosclerosis.

5. The remedy for ischemic disease according to claim 1, which is used for obtaining a necessary and adequate amount of hematopoietic stem cells from the bone marrow to treat ischemic disease by administration of autologous hematopoietic stem cells.

6. The remedy for ischemic disease according to claim 2, which is used for obtaining a necessary and adequate amount of hematopoietic stem cells from the bone marrow to treat trauma, rejection reaction during transplantation, ischemic cerebrovascular disorder, ischemic renal disease, ischemic pulmonary disease, infection-related ischemic disease, ischemic disease of limbs or ischemic heart disease by administration of autologous hematopoietic stem cells.

7. The remedy for ischemic disease according to claim 3, which is used for obtaining a necessary and adequate amount of hematopoietic stem cells from the bone marrow to treat apoplexy, cerebral infarction, ischemic cardiomyopathy, myocardial infarction, ischemic heart failure or obstructive arteriosclerosis by administration of autologous hematopoietic stem cells.

8. The remedy for ischemic disease according to claim 4, which is used for obtaining a necessary and adequate amount of hematopoietic stem cells from the bone marrow to treat obstructive arteriosclerosis by administration of autologous hematopoietic stem cells.

9. The remedy for ischemic disease according to claim 1, which is used for obtaining a necessary and adequate amount of hematopoietic stem cells from the peripheral blood to treat ischemic disease by administration of autologous hematopoietic stem cells.

10. The remedy for ischemic disease according to claim 2, which is used for obtaining a necessary and adequate amount of hematopoietic stem cells from the peripheral blood to treat trauma, rejection reaction during transplantation, ischemic cerebrovascular disorder, ischemic renal disease, ischemic pulmonary disease, infection-related ischemic disease, ischemic disease of limbs or ischemic heart disease by administration of autologous hematopoietic stem cells.

11. The remedy for ischemic disease according to claim 3, which is used for obtaining a necessary and adequate amount of hematopoietic stem cells from the peripheral blood to treat apoplexy, cerebral infarction, ischemic cardiomyopathy, myocardial infarction, ischemic heart failure or obstructive arteriosclerosis by administration of autologous hematopoietic stem cells.

12. The remedy for ischemic disease according to claim 4, which is used for obtaining a necessary and adequate amount of hematopoietic stem cells from the peripheral blood to treat obstructive arteriosclerosis by administration of autologous hematopoietic stem cells.

13. The remedy for ischemic disease according to any one of claims 1 to 4, wherein the hematopoietic stem cells administered and hence increased in the peripheral blood contribute to vasculogenesis in a diseased part.

14. A method for treating ischemic disease, wherein a therapy for ischemic disease comprising administering to a subject a factor having an angiogenic action or a gene thereof is used in combination with a remedy for ischemic disease comprising granulocyte colony-stimulating factor as an active ingredient.

15. The method for treating ischemic disease according to claim 14, wherein the factor having an angiogenic action is hepatocyte growth factor or fibroblast growth factor.

16. A method for treating obstructive arteriosclerosis, wherein a therapy for obstructive arteriosclerosis comprising administering a factor having an angiogenic action or a gene thereof at a site near a diseased part is used in combination with a remedy for ischemic disease comprising granulocyte colony-stimulating factor as an active ingredient.

17. The method for treating obstructive arteriosclerosis according to claim 16, wherein the factor having an angiogenic action is hepatocyte growth factor or fibroblast growth factor.

18. A method for treating ischemic disease, wherein the remedy for ischemic disease according to any one of claims 1 to 3 is used in combination with a drug clinically used as a pharmacotherapy for ischemic disease, such as an antiplatelet agent, a vasodilator, a microcirculation improver, an anticoagulant or an antilipemic agent.

19. A method for treating obstructive arteriosclerosis, wherein the remedy for ischemic disease according to claim 4 is used in combination with a drug clinically used as a pharmacotherapy for obstructive arteriosclerosis, such as an antiplatelet agent, a vasodilator, a microcirculation improver, an anticoagulant or an antilipemic agent.

20. The use of granulocyte colony-stimulating factor and hepatocyte growth factor or fibroblast growth factor for the treatment of ischemic disease.

21. The use according to claim 20, wherein the ischemic disease is trauma, rejection reaction during transplantation, ischemic cerebrovascular disorder, ischemic renal disease, ischemic pulmonary disease, infection-related ischemic disease, ischemic disease of limbs or ischemic heart disease.

22. The use according to claim 20, wherein the ischemic disease is apoplexy, cerebral infarction, ischemic cardiomyopathy, myocardial infarction, ischemic heart failure or obstructive arteriosclerosis.

23. The use according to claim 20, wherein the ischemic disease is obstructive arteriosclerosis.

24. The use of granulocyte colony-stimulating factor and hepatocyte growth factor or fibroblast growth factor for obtaining a necessary and adequate amount of hematopoietic stem cells from the bone marrow to treat ischemic disease by administration of autologous hematopoietic stem cells.

25. The use according to claim 24, wherein the ischemic disease is trauma, rejection reaction during transplantation, ischemic cerebrovascular disorder, ischemic renal disease, ischemic pulmonary disease, infection-related ischemic disease, ischemic disease of limbs or ischemic heart disease.

26. The use according to claim 24, wherein the ischemic disease is apoplexy, cerebral infarction, ischemic cardiomyopathy, myocardial infarction, ischemic heart failure or obstructive arteriosclerosis.

27. The use according to claim 24, wherein the ischemic disease is obstructive arteriosclerosis.

28. The use of granulocyte colony-stimulating factor and hepatocyte growth factor or fibroblast growth factor for obtaining a necessary and adequate amount of hematopoietic stem cells from the peripheral blood to treat ischemic disease by administration of autologous hematopoietic stem cells.

29. The use according to claim 28, wherein the ischemic disease is trauma, rejection reaction during transplantation, ischemic cerebrovascular disorder, ischemic renal disease, ischemic pulmonary disease, infection-related ischemic disease, ischemic disease of limbs or ischemic heart disease.

30. The use according to claim 28, wherein the ischemic disease is apoplexy, cerebral infarction, ischemic cardiomyopathy, myocardial infarction, ischemic heart failure or obstructive arteriosclerosis.

31. The use according to claim 28, wherein the ischemic disease is obstructive arteriosclerosis.

32. The use of granulocyte colony-stimulating factor and hepatocyte growth factor or fibroblast growth factor in a therapy for ischemic disease comprising administering to a subject a factor having an angiogenic action or a gene thereof.

33. The use according to claim 32, wherein the ischemic disease is trauma, rejection reaction during transplantation, ischemic cerebrovascular disorder, ischemic renal disease, ischemic pulmonary disease, infection-related ischemic disease, ischemic disease of limbs or ischemic heart disease.

34. The use according to claim 32, wherein the ischemic disease is apoplexy, cerebral infarction, ischemic cardiomyopathy, myocardial infarction, ischemic heart failure or obstructive arteriosclerosis.

35. The use of granulocyte colony-stimulating factor and hepatocyte growth factor or fibroblast growth factor in a therapy for obstructive arteriosclerosis comprising administering a factor having an angiogenic action or a gene thereof at a site near a diseased part.
